# EUROPEAN PATENT APPLICATION

(11) **EP 4 741 816 A1**
(43) Date of publication of application: **13.05.2026**
(21) Application number: 24211720.8
(22) Date of filing: 08.11.2024
(51) Int. Cl.: G01N 29/14, G01N 29/24, G01N 33/38

(54) **REMOTE SUFACE ACOUSTIC WAVE SENSING OF CURABLE CHEMICAL MORTAR SYSTEMS FOR CHEMICAL FASTENING**

(71) Applicant: Hilti Aktiengesellschaft, 9494 Schaan (LI)
(72) Inventor: Pfeil, Armin, 86899 Landsberg am Lech (DE)
(74) Representative: Hilti Aktiengesellschaft Corporate Intellectual Property

(57) **Abstract**

The present invention relates to a surface acoustic wave sensing system comprising a surface acoustic wave sensor configured to be provided relative to a curable chemical mortar system and to output, to a measurement device or system, data corresponding to characteristics of the curable chemical mortar system identifying whether the curable chemical mortar system is curing correctly, data for in service control after curing and/or data corresponding to characteristics of the chemical mortar system associated with use of the chemical mortar system when installed.

## Description

### Field of the invention

The present invention relates to remote surface acoustic wave sensing of characteristics of curable chemical systems, in particular curable chemical mortar injection systems in the field of chemical fastening.

### Background of the invention

Many mortar systems exist which provide a good chemical fastening of anchors and post-installed reinforcing bars in mineral substrates or surfaces. For example, organic systems based on free-radically polymerizable resins are used when fast curing is desired, inorganic systems are used when it comes to desired fire performance and slow curing systems exists when a longer installation time is needed. In particular, curable chemical mortar injection systems are directly employed on the job site, thereby being introduced into borehole and then left to cure with the appropriate anchoring means embedded into the mineral substrate of surface.

In order to achieve a safe and fully load bearing system the curing of the chemical mortar injection system is required that always involves monitoring the curing time which is strongly dependent on the installation temperature, material installed and on specific weather conditions surrounding the job site. This monitoring is often time-consuming and inaccurate and may result in not fully cured system which in the end is unsafe to use or in a worst-case scenario cannot bear the fully load.

There are known systems for identifying and tracking different objects which involve use of radio frequency identification devices. However, radio frequency identification devices can only be employed in a distance range very close to the embedded RFID chip which is usually in the range of some millimetres. They can further only be used due to their sensitivity in safe environments and need a large number of tags in order to establish a full picture of the monitored system. In parallel it is only possible to read 1 to 3 tags at the time. The RFID chip technology, however, is not suitable for monitoring certain conditions, which are based on a physical property of an object or material.

When it comes to chemical fastening of anchoring means monitoring of the curing process and the fully cured system, also under harsh conditions is needed to ensure provision of a safe system on the jobsite, thereby also safing labour time.

Therefore, there is a need for a monitoring system for curable chemical systems, in particular curable chemical mortar injection systems, in the field of chemical fastening, which is superior over the prior art systems with regard to health and safety, handling, labor time, sustainability and a good balance between setting and hardening of the mortar as well as maintain the cured system. Moreover, it is of interest to provide a system that can be used for monitoring chemical fastening of anchoring means in mineral substrates without adversely affecting the handling, characteristics and the mechanical performance of the chemical anchoring system as well as to provide a system that is constantly monitored to ensure that a decrease of performance over time is not happening. Finally, there is a need for a system that enables the customer to refrain from referring to curing timetables in order to determine the final installation state of a cured chemical fastening system.

It would be beneficial if the chemical mortar injection system can remotely send out information about its curing.

In view of the above, it is an object of the present invention to provide a monitoring system for curable chemical systems, in particular curable chemical mortar injection systems, in the field of chemical fastening, which overcomes the disadvantages of the prior art systems. In particular, it is an object to provide a surface acoustic wave sensing system, which can be handled easily and is eco-friendly, sustainable, can be used under harsh conditions and over a wide distance range and that has a well balanced parallel readability of sensors.

Moreover, it is an object of the present invention to provide method for remote surface acoustic wave sensing of curable chemical mortar systems as well as employing a handheld device in said method.

These and other objectives as they will become apparent from the ensuring description of the invention are solved by the present invention as described in the independent claims. The dependent claims pertain to preferred embodiments.

### Summary of the invention

One aspect of the present invention relates to a surface acoustic wave sensing system comprising a surface acoustic wave sensor configured to be provided relative to a curable chemical mortar system and to output, to a measurement device or system, data corresponding to characteristics of the curable chemical mortar system identifying whether the curable chemical mortar system is curing correctly, data for in service control after curing and/or data corresponding to characteristics of the chemical mortar system associated with use of the chemical mortar system when installed.

Another aspect of the present invention relates to a method for remote surface acoustic wave sensing of curable chemical mortar systems as well as employing a handheld device in said method.

### Description of the invention

As used in the context of present invention, the singular forms of "a" and "an" also include the respective plurals unless the context clearly dictates otherwise. Thus, the term "a" or "an" is intended to mean "one or more" or "at least one", unless indicated otherwise.

Surface acoustic wave sensing technology may be used as the new low-cost technology for continuously monitoring and establishing of real-time awareness of operating conditions in complex systems. Surface acoustic waves have been used for filtering, for example in cellular phones, as well as for sensing, such as in industrial loT sensors. Wired surface acoustic wave chemical sensors are commercially employed in nerve and blister agent detection and are often coupled to GC systems. Recently wireless surface acoustic wave sensors have been improved in the field of communication. Surface acoustic wave sensing technology is able to make measurements such as strain, vibration, humidity, voltage, switch position, corrosion, pressure, strain, torque, temperature, viscosity and mass.

Surface acoustic wave sensors and tags have the follow following advantages: they can operate wirelessly, thereby eliminating wiring, they reduce installation costs, they can operate on rotating parts and spread spectrum communications. Further, they are RFID capable, whereby multiple sensors can be read at once, i.e. up to 100 acoustic responses; they require no batteries, hence no batterie change is needed, thereby reducing maintenance costs, they can be used in hard to access locations.

Surface acoustic wave sensors and tags are sensitive and accurate comparable to wired sensors. Moreover, they are able to perform real- time measurements whereby a rapid response times allows their use for real-time process control and monitoring. Surface acoustic wave sensors have a very long lifetime and hence are suitable for embedded use as well as long term monitoring. Finally, surface acoustic wave sensors and tags survive and operate in extreme conditions, such as at elevated temperatures, in high humidity areas or under very cold conditions.

When it comes to chemical fastening of anchoring means in mineral substrates, it has been surprisingly found out that the employment of surface acoustic waves to a curable chemical mortar system enables real-time monitoring and establishing real-time conditions in curable chemical mortar systems, by measuring pressure, strain, torque, temperature, and mass, thereby providing a system that addresses the disadvantages of the prior art systems.

In particular, for curing monitoring, the change of the dampening of the chemical injection mortar is measured by its increasing stiffness during curing. It has been found that in the field of chemical fastening of anchoring means using a curable chemical injection mortar system, monitoring dampening and hence viscosity is crucial. The higher it is, the less the acoustic waves near the surface of the sensor are attenuated, and if these signals no longer change significantly and exceed a limit known from calibration measurements, curing is complete.

Further, for structural health monitoring of installed anchors, compressive strength, deformation and softening can be monitored indicating occurrence of a loss in compressive strength, creep, and/or degradation thereby pointing to a critical behavior of the monitored system.

The surface acoustic wave sensing system according to the present invention comprises a surface acoustic wave sensor configured to be provided relative to a curable chemical mortar system and to output, to a measurement device or system, data corresponding to characteristics of the curable chemical mortar system identifying whether the curable chemical mortar system is curing correctly, data for in service control after curing and/or data corresponding to characteristics of the chemical mortar system associated with use of the chemical mortar system when installed.

In particular, the surface acoustic wave sensor may be provided on the anchoring means, such a bolts, rods, reinforcement bars, screws, at the interface of the chemical mortar to mineral substrate, such as concrete, or at different embedment depths.

It is also possible to provide more than one surface acoustic wave sensor in the surface acoustic wave sensing system, which can be read at the same time, giving more comprehensive information about the chemical mortar system compared to a single-sensor solution.

The surface acoustic wave sensing system of the present invention is a system, that utilizes a passive remote surface acoustic wave sensor. The sensors can therefore remain during curing and in the final cured chemical mortar system and hence can be used to deliver information about condition characteristics of the chemical mortar system also during lifetime.

According to the present invention is the surface acoustic wave sensor configured to receive an input signal from a measurement device or system to generate an output signal to a measurement device or system. In particular, the surface acoustic wave sensor is configured to output from the curable chemical mortar system to a measurement device or system only the data corresponding to characteristics of the curable chemical mortar system to identify whether the curable chemical mortar system is curing or installed correctly or is no longer curing or installed correctly. Further, data corresponding to characteristics of the curable chemical mortar system associated with use of the cured chemical mortar system when installed can be outputted by the surface acoustic wave sensor. Additionally, product-IDs, batch-IDs can be transmitted.

The input signal applied in the present invention is a radio wave signal, preferably with an operating frequency of 2.4 GHz. The output signal is a radio frequency signal corresponding to the condition characteristics of the chemical mortar system.

The data generated by the output signal includes data corresponding to one or more of a temperature associated with the curable chemical mortar system, stress and strains placed in or on the curable chemical mortar system, a compressive force applied to the curable chemical mortar system, pressure, movement, resistance and a unique identifier that identifies the curable chemical mortar system. Also, data corresponding to parameters such as humidity, creep, softening, deformation, loss of pre-tension and/or torque can be transmitted.

The surface acoustic wave sensing system according to the present invention further comprises a measurement device or system. Preferably the measurement device or system is a handheld device.

The present invention allows monitoring of a curable chemical mortar system, either during curing process and/or in a fully cured state. The curable chemical mortar system may be an organic or inorganic curable chemical mortar injection system used for chemical fastening of anchoring means in mineral substrates. This method for remote surface acoustic wave sensing of curable chemical mortar system comprises generating an input signal with a measurement device or system; applying the input signal to a surface acoustic wave sensor thereby passing a sensing region of the sensor; generating an output signal, and receiving the output signal with a measurement device or system. After receiving the output signal, it can be evaluated.

The measurement device or system, in particular the surface acoustic wave reader, can be communicably connected to a communication interface, and can receive data from the communication interface corresponding to the feedback regarding the characteristics of the chemical mortar system. A processor which may be implemented in circuitry, software or a combination of the two, can perform analysis on the data to determine information pertaining to curing and installation of the chemical mortar system. For example, the processor can analyze the received data to determine whether the chemical mortar system has been installed correctly (or incorrectly) and/or is no longer installed correctly based on a comparison of the received data to predetermined or known data regarding correct (or incorrect) installation. As non-limiting examples, the predetermined or known data regarding correct (or incorrect) installation of the chemical mortar system can include stress, strain, pressure, or force thresholds for chemical mortar system.

As another example, the measurement device or system can analyze the received data to determine whether characteristics of the chemical mortar system pertaining to use or operating conditions of the chemical mortar system, when installed, are within acceptable predefined limits or thresholds.

Examples of predefined limits or thresholds for acceptable use of the chemical mortar system can include, but are not limited to, limits or thresholds involving a temperature associated with the chemical mortar system; presence of water in the chemical mortar system; air flow or cracks associated with the chemical mortar system; stress, pressure and/or strain placed in or on the chemical mortar system or provided by the chemical mortar system; dampening and a compressive force applied to or by the chemical mortar system.

The predetermined or known data and/or the predefined limits or thresholds can be retrieved from memory of the measurement device or system. Further, the predetermined or known data and/or the predefined limits or thresholds can be input using the user interface. The user interface may include a display configured to output information, based on the analysis, pertaining to installation of the chemical mortar system and/or to determine whether characteristics of the chemical mortar system pertaining to use or operating conditions of the chemical mortar system when installed are within acceptable predefined limits.

Also, in one or more embodiments, the measurement device or system may also be a subcomponent of a multi-function device, such as a portable electronic communication device, including a smart device (e.g., a cell phone, PDA, tablet, etc.). Alternatively, the measurement device or system may be a standalone device. It can also be a mobile smart device and can additionally be coupled with a radio frequency identification (RFID) interrogator, loT or BIM interface. Additionally or alternatively, the measurement device or system may be a so-called "back office" measurement device or system that can monitor one or more chemical mortar systems.

The present invention shows a use of a remote passive surface acoustic wave sensor in a method for remote sensing of curable chemical mortar systems for chemical fastening of anchoring means in mineral substrates, in particular for a curable chemical mortar system, wherein the curable chemical mortar system is an organic or inorganic curable chemical mortar injection system.

The remote generating of the input signal and remote receiving the output signal, allows for a remote operation over a distance of several meters. Using a remote passive surface acoustic wave sensor, allows for a system that can be handled easily and is eco-friendly, sustainable, can be used under harsh conditions and over a wide distance range and that has a well balance parallel readability of sensors even under unfavorable conditions.

While aspects of the present invention have been particularly described with reference to the embodiments above, it will be understood by those skilled in the art that various additional embodiments may be contemplated by the modification of the disclosed systems and methods without departing from the spirit and scope of what is disclosed. Such embodiments should be understood to fall within the scope of the present invention as determined based upon the claims and any equivalents thereof.

## Claims

1. A surface acoustic wave sensing system comprising a surface acoustic wave sensor configured to be provided relative to a curable chemical mortar system and to output, to a measurement device or system, data corresponding to characteristics of the curable chemical mortar system identifying whether the curable chemical mortar system is curing correctly, data for in service control after curing and/or data corresponding to characteristics of the chemical mortar system associated with use of the chemical mortar system when installed.

2. Surface acoustic wave sensing system according to claim 1, wherein the surface acoustic wave sensor is configured to receive an input signal from a measurement device or system to generate the output signal to a measurement device or system.

3. Surface acoustic wave sensing system according to claim 1 or 2, wherein the surface acoustic wave sensor is a passive remote surface acoustic wave sensor.

4. Surface acoustic wave sensing system according to any one of the preceding claims, wherein the surface acoustic wave sensor is configured to output from the curable chemical mortar system to a measurement device or system only the data corresponding to characteristics of the curable chemical mortar system to identify whether the curable chemical mortar system is curing or installed correctly or is no longer curing or installed correctly.

5. Surface acoustic wave sensing system according to any one of the preceding claims, wherein the surface acoustic wave sensor is configured to output from the curable chemical mortar system to a measurement device or system only the data corresponding to characteristics of the curable chemical mortar system associated with use of the cured chemical mortar system when installed.

6. Surface acoustic wave sensing system to any one of the preceding claims, wherein the data corresponding to characteristics of the curable chemical mortar system associated with use of the curable chemical mortar system when installed include data corresponding to one or more of a temperature associated with the curable chemical mortar system, stress and strains placed in or on the curable chemical mortar system, a compressive force applied to the curable chemical mortar system, pressure, movement, resistance and a unique identifier that identifies the curable chemical mortar system.

7. Surface acoustic wave sensing system according to any one of the preceding claims, further comprising a measurement device or system.

8. Surface acoustic wave sensing system according to 7, wherein the measurement device or system is a handheld device.

9. Surface acoustic wave sensing system according to any one of the preceding claims, wherein the curable chemical mortar system is an organic or inorganic curable chemical mortar injection system.

10. Surface acoustic wave sensing system according to any one of the preceding claims, wherein the curable chemical mortar system is an organic or inorganic curable chemical mortar injection system used for chemical fastening of anchoring means in mineral substrates.

11. Surface acoustic wave sensing system according to any one of claims 2 to 10, wherein the input signal is a radio wave signal with an operating frequency of 2.4 GHz.

12. Surface acoustic wave sensing system according to any one of claims 2 to 11, wherein the output signal is a radio frequency signal corresponding to the condition characteristics of the chemical mortar system.

13. Method for remote surface acoustic wave sensing of curable chemical mortar system comprising:
generating an input signal with a measurement device or system;
applying the input signal to a surface acoustic wave sensor thereby passing a sensing region of the sensor;
generating an output signal, and
receiving the output signal with a measurement device or system.

14. The method according to claim 13, further comprising evaluating the output signal.

15. The method according to claim 13 or 14, wherein the measurement device or system is a handheld device.

16. Use of a remote passive surface acoustic wave sensor in a method for remote sensing of curable chemical injection mortar systems for chemical fastening of anchoring means in mineral substrates.
